# EUROPÄISCHE PATENTSCHRIFT

(11) **EP 0 471 987 B1**
(45) Veröffentlichungstag und Bekanntmachung des Hinweises auf die Patenterteilung: **05.01.1994**
(21) Anmeldenummer: 91112153.1
(22) Anmeldetag: 20.07.1991
(51) Int. Cl.: C07C 19/045, C07C 17/02

(54) **Verfahren und Vorrichtung zur Herstellung von hochreinem 1,2-Dichlorethan mit Wärmerückgewinnung**
Process and apparatus for the preparation of very pure 1,2-dichlorethane with heat recovery
Procédé et dispositif pour la préparation de 1,2-dichloréthane très pur avec une récupération de chaleur

(30) Priorität: 20.08.1990 DE 4026282
(43) Veröffentlichungstag der Anmeldung: 26.02.1992
(73) Patentinhaber: HOECHST AKTIENGESELLSCHAFT, 65926 Frankfurt am Main (DE)
(72) Erfinder: Rechmeier, Gerhard, Dr., W-5042 Erftstadt (DE)

(56) Entgegenhaltungen:
- EP-A- 0 080 098
- EP-A- 0 111 203
- DE-C- 3 445 896

## Beschreibung

Die Erfindung betrifft ein Verfahren zur Herstellung von hochreinem 1,2-Dichlorethan mit Wärmerückgewinnung durch Umsetzung von Ethylen und Chlor in flüssigem 1,2-Dichlorethan in Gegenwart eines speziellen Katalysators, wobei unerwünschte andere chlorierte Produkte nur in unwesentlichen Mengen entstehen, so daß das gebildete 1,2-EDC nicht destillativ gereinigt werden muß. Eine Vorrichtung zur Durchführung des Verfahrens wird angegeben.

Die katalytische Anlagerung von Chlor an Ethylen in 1,2-Dichlorethan als Lösemittel verläuft in 3 Stufen:
1. Lösung des gasförmigen Chlors in 1,2-Dichlorethan
2. Lösung des gasförmigen Ethylens in 1,2-Dichlorethan
3. Reaktion der gelösten Reaktanten zu 1,2-Dichlorethan.

Während die Stufen 1 und 3 schnell ablaufen, verläuft die Stufe 2, wahrscheinlich wegen der nur geringen Löslichkeit von Ethylen in 1,2-Dichlorethan, langsam und ist deshalb die geschwindigkeitsbestimmende Reaktionsstufe. Aus diesem Grund erfolgte in der Technik bisher die 1,2-Dichlorethan-Herstellung in Reaktoren, die aus einem Hauptreaktor, mit ausreichender Verweilzeit, und einem Nachreaktor, zur vollständigen Umsetzung des Ethylens, bestanden.

Nach dem Vorschlag der EP-0 080 098 B1 (CA-1 221 708) werden Chlor und Ethylen gasförmig in 1,2-Dichlorethan als Lösemittel in einem Doppel-Schlaufenreaktor mit Nachreaktor umgesetzt. Im Hauptreaktor ist zur Feinverteilung der Reaktionsgase eine mit Füllkörpern beschickte Mischzone angeordnet. Die Reaktionszeit ist mit 1 bis 15 Stunden angegeben. Die Reinigung des 1,2-Dichlorethans erfolgt in nachgeschalteten Destillationskolonnen.

In der DE-C-34 45 896 wird zur Herstellung von 1,2-Dichlorethan ein Static-Mischer oder eine Strahl-Düse zur Dispergierung von gäsförmigem Ethylen im flüssigen 1,2-Dichlorethan-Kreislauf vorgeschlagen.

Für die 1,2-Dichlorethan-Herstellung wird in der EP-0 111 203 A1 (US-A-4.774.373) ein spezieller Katalysator angegeben, bei dessen Einsatz nur eine geringe Korrosionsrate an den Stahloberflächen der Vorrichtungsteile resultiert. Dieser spezielle Katalysator besteht aus wasserfreiem Tetrachloroferrat (1-), dessen Kation ein Alkali-, Erdalkalimetall- oder ein Ammoniumion ist.

Es bestand somit die Aufgabe, die bekannten Arbeitsweisen und Vorrichtungen in dem Maße zu verändern, daß die Bildung der gesamten chlorierten Nebenprodukte im 1,2-Dichlorethan unter einem Wert von 500 ppm liegt und sowohl die Installation eines großen Haupt- und Nebenreaktors als auch eine destillative Nebenproduktabtrennung entfallen und die Reaktionsenthalpie in variabler Weise für andere Verfahren vollständig zur Verfügung gestellt wird.

Gegenstand der Erfindung ist somit ein Verfahren zur Herstellung von hochreinem 1,2-Dichlorethan mit Wärmerückgewinnung aus äquimolaren Mengen von Ethylen und Chlor in 1,2-Dichlorethan als Lösemittel in Gegenwart eines Tetrachloroferrat(1-)-Katalysators bei einer Temperatur von 75 bis 200 °C und einem Druck von 1 bis 15 bar in einer Reaktionszone, welches dadurch gekennzeichnet ist, daß man in einer vorgeschalteten Mischzone das Chlorgas im umlaufenden 1,2-Dichlorethan löst, in der nachgeschalteten Reaktionszone das Ethylengas in feindisperser flüssiger Phase mit einem Blasendurchmesser von maximal 2,0 mm umsetzt, diese feindisperse flüssige Phase mit einer Geschwindigkeit von 0,3 bis 1 m/s bei einer Verweildauer von 2,5 bis 25 Sekunden, berechnet auf die flüssige Phase, die Reaktionszone durchströmen läßt, und danach das gebildete hochreine 1,2-Dichlorethan, welches weniger als 500 ppm chlorierte Nebenprodukte enthält, über eine Entspannungsverdampfung gasförmig abzieht.

Das Verfahren kann weiterhin wahlweise noch dadurch gekennzeichnet sein, daß der Blasendurchmesser des Ethylengases kleiner als 1,5 mm ist.

Mit dem erfindungsgemäßen Verfahren wird nunmehr ein sehr einfaches Verfahren zur Herstellung von hochreinem 1,2-Dichlorethan angegeben. Nach einer Entspannungsverdampfung wird das gasförmige 1,2-Dichlorethan kondensiert und fällt in einer Reinheit von mehr als 99,95 Gew.-% an. Es enthält als wesentlichen Nebenbestandteil 1,1,2-Trichlorethan. Die bei der Kondensation freigesetzte Wärme kann beispielsweise zur Destillation von 1,2-Dichlorethan, welches nach dem Oxichlorierungsverfahren erhalten wird, genutzt werden. Die sonst bei der 1,2-Dichlorethan-Herstellung nach dem Direktchlorierungsverfahren benötigten Destillationskolonnen zur Leichtsieder- und Hochsiederabtrennung entfallen bei dem erfindungsgemäßen Verfahren vollständig. Dadurch werden sowohl die entsprechenden Investitionskosten als auch Betriebskosten eingespart. Die Ausbeuten an 1,2-Dichlorethan betragen 99,7 %, bezogen auf C₂H₄, und 99,7 %, bezogen auf Cl₂.

Für das erfindungsgemäße Verfahren ist die Feinverteilung des Ethylengases von ausschlaggebender Bedeutung. Es reicht nicht aus, das Ethylengas in feinverteilter Form in das 1,2-Dichlorethan einzudüsen, da die Ethylengasbläschen schnell zu großen Blasen zusammenwachsen und dann die Reaktionsgeschwindigkeit stark absinkt. Deshalb ist dafür Sorge zu tragen, daß die Vereinigung zu größeren Ethylengasblasen verhindert wird. Diese Feinverteilung des Ethylengases wird in sogenannten "Statischen Mischern" erreicht, wie sie in Chemie-Ingenieur-Technik 52 (1980), Nr. 4, Seiten 285 bis 291, beschrieben sind.

Es wird weiter eine Vorrichtung zur Durchführung des Verfahrens angegeben, die gekennzeichnet ist durch einen Mischer 1 mit Chloreinleitung 11, einen "Statischen Mischer" 2 mit Ethyleneinleitung 5, ein Entspannungsgefäß 3, eine Umwälzpumpe 6 und einen Wärmetauscher 7, wobei der Mischer 1, der "Statische Mischer" 2, das Entspannungsgefäß 3, die Umwälzpumpe 6 und der Wärmetauscher 7 strömungsmäßig miteinander verbunden sind, das Entspannungsgefäß 3 über eine Drossel 4 mit einem Kondensator 8 verbunden ist und vom Kondensator 8 eine Produktabflußleitung 9 sowie eine Abgasleitung 10 abgehen.

Die Vorrichtung kann wahlweise noch dadurch gekennzeichnet sein, daß
a) ein "Statischen Mischer" 2 mit schräger Stegumströmung eingesetzt wird,
b) die Bleche im "Statischen Mischer" 2 gelocht, geschlitzt, gezackt und/oder gewellt sind,
c) die Bleche mäandernde Kanäle oder sich kreuzende Kanäle bilden,
d) zwischen dem "Statischen Mischer" 2 und dem Entspannungsgefäß 3 eine Druckhaltung 12 angeordnet ist,
e) anstelle der Druckhaltung 12 das Entspannungsgefäß 3 8 bis 12 m oberhalb des "Statischen Mischers" 2 angeordnet ist.

Der auf diese Weise im "Statischen Mischer" 2 erhöhte Druck verhindert ein Sieden des 1,2-Dichlorethans in der Reaktionszone, wodurch eine Verringerung der Reaktionsgeschwindigkeit eintreten würde.

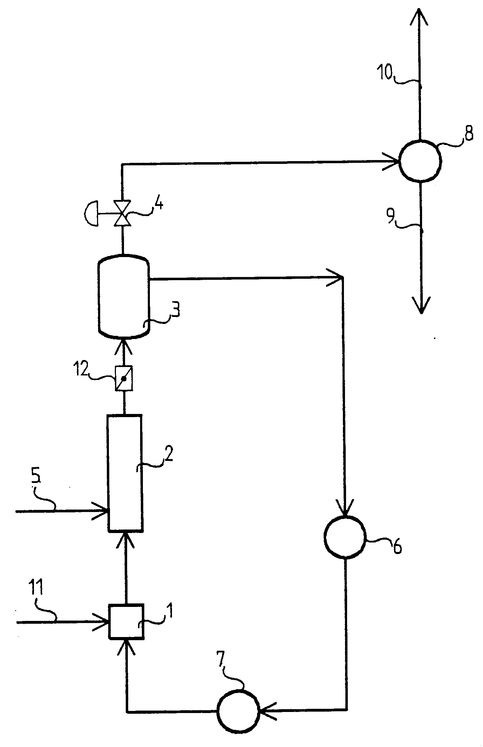

Das Verfahren und die Vorrichtung zur Herstellung von hochreinem 1,2-Dichlorethan werden im folgenden anhand der beiliegenden Zeichnung näher erläutert.

Das Entspannungsgefäß 3, der Wärmetauscher 7, die Mischzone 1 sowie der "Statische Mischer" 2 werden mit 1,2-Dichlorethan gefüllt und mit der Umwälzpumpe 6 wird ein 1,2-Dichlorethanstrom von 200 m³/h eingestellt. Durch die Leitung 11 werden 2867 kg/h Chlorgas in die Mischzone 1 dosiert. Die Mischzone 1 ist in der Art einer Mischdüse ausgebildet. Das mit Chlor beladene 1,2-Dichlorethan enthält 0,2 Gew.-% NaFeCl₄ als Katalysator und strömt in den "Statischen Mischer" 2, in den durch Leitung 5 1134 kg/h Ethylengas dosiert werden. Als "Statischer Mischer" 2 wird ein 3 m langer SMV-Mischer der Firma Sulzer, Winterthur/Schweiz, mit gewellten Blechen, die offene, sich kreuzende Kanäle bilden, eingesetzt.

Der Ethylengasblasendurchmesser betrug 1,0 bis 1,5 mm; das 1,2-Dichlorethan durchströmt den "Statischen Mischer" 2 mit einer Geschwindigkeit von 0,4 m/s.

Nach Einstellen des stationären Zustandes im Kondensator 8 werden 7,5 m³/h Wasser auf 60 °C erwärmt; im Wärmetauscher 7 werden weitere 20,0 m³/h Wasser auf 96 °C erwärmt. Der 1,2-Dichlorethankreislauf wird bei 125 °C und einem Druck von 4 bar betrieben. Aus dem Entspannungsgefäß 3 wird über eine Standregelung das gebildete 1,2-Dichlorethan durch die Drossel 4 gasförmig in den Kondensator 8 geleitet. Stündlich werden 3997 kg 1,2-Dichlorethan durch den Dichlorethanabfluß 9 abgezogen. Durch die Abgasleitung 10 werden nichtkondensierbare Gase zu einer Verbrennung geleitet.

Das produzierte 1,2-Dichlorethan enthält als chlorierte Verunreinigungen noch:
400 ppm 1,1,2-Trichlorethan
50 ppm 1,1-Dichlorethan
5 ppm 1,2-Dichlorethylen-trans
- Die Ausbeute beträgt: 99,7 %, bezogen auf C₂H₄
99,7 %, bezogen auf Cl₂.

## Patentansprüche

1. Verfahren zur Herstellung von hochreinem 1,2-Dichlorethan mit Wärmerückgewinnung aus äquimolaren Mengen von Ethylen und Chlor in 1,2-Dichlorethan als Lösemittel in Gegenwart eines Tetrachloroferrat(1-)-Katalysators bei einer Temperatur von 75 bis 200 °C und einem Druck von 1 bis 15 bar in einer Reaktionszone, dadurch gekennzeichnet, daß man in einer vorgeschalteten Mischzone das Chlorgas im umlaufenden 1,2-Dichlorethan löst, in der nachgeschalteten Reaktionszone das Ethylengas in feindisperser flüssiger Phase mit einem Blasendurchmesser von maximal 2,0 mm umsetzt, diese feindisperse flüssige Phase mit einer Geschwindigkeit von 0,3 bis 1 m/s bei einer Verweildauer von 2,5 bis 25 Sekunden, berechnet auf die flüssige Phase, die Reaktionszone durchströmen läßt, und danach das gebildete hochreine 1,2-Dichlorethan, welches weniger als 500 ppm chlorierte Nebenprodukte enthält, über eine Entspannungsverdampfung gasförmig abzieht.

2. Verfahren nach Anspruch 1, dadurch gekennzeichnet, daß der Blasendurchmesser des Ethylengases kleiner als 1,5 mm ist.

3. Vorrichtung zur Durchführung des Verfahrens nach einem der Ansprüche 1 oder 2, gekennzeichnet durch einen Mischer (1) mit Chloreinleitung (11), einen "Statischen Mischer" (2) mit Ethyleneinleitung (5), ein Entspannungsgefäß (3), eine Umwälzpumpe (6) und einen Wärmetauscher (7), wobei der Mischer (1), der "Statische Mischer" (2), das Entspannungsgefäß (3), die Umwälzpumpe (6) und der Wärmetauscher (7) strömungsmäßig miteinander verbunden sind, das Entspannungsgefäß (3) über eine Drossel (4) mit einem Kondensator (8) verbunden ist und vom Kondensator (8) eine Produktabflußleitung (9) sowie eine Abgasleitung (10) abgehen.

4. Vorrichtung nach Anspruch 3, dadurch gekennzeichnet, daß ein "Statischer Mischer" (2) mit schräger Stegumströmung eingesetzt wird.

5. Vorrichtung nach Anspruch 4, dadurch gekennzeichnet, daß die Bleche im "Statischen Mischer" (2) gelocht, geschlitzt, gezackt und/oder gewellt sind.

6. Vorrichtung nach Anspruch 5, dadurch gekennzeichnet, daß die Bleche mäandernde Kanäle bilden.

7. Vorrichtung nach Anspruch 5, dadurch gekennzeichnet, daß die Bleche sich kreuzende Kanäle bilden.

8. Vorrichtung nach Anspruch 3, dadurch gekennzeichnet, daß zwischen dem "Statischen Mischer" (2) und dem Entspannungsgefäß (3) eine Druckhaltung (12) angeordnet ist.

9. Vorrichtung nach Anspruch 3, dadurch gekennzeichnet, daß das Entspannungsgefäß (3) 8 bis 12 m oberhalb des "Statischen Mischers" (2) angeordnet ist.

## Claims

1. A process for preparing high-purity 1,2-dichloroethane with heat recovery from equimolar amounts of ethylene and chlorine in 1,2-dichloroethane as solvent in the presence of a tetrachloroferrate(1⁻) catalyst at a temperature of 75 to 200°C and a pressure of 1 to 15 bar in a reaction zone, which process comprises dissolving the chlorine gas in recirculating 1,2-dichloroethane in an upstream mixing zone, reacting the ethylene gas in finely disperse liquid phase having a bubble diameter of at most 2.0 mm in the downstream reaction zone, allowing this finely disperse liquid phase to flow through the reaction zone at a rate of 0.3 to 1 m/s with a residence time of 2.5 to 25 seconds, calculated relative to the liquid phase, and then removing the high-purity 1,2-dichloroethane formed, which contains less than 500 ppm of chlorinated byproducts, in gaseous form by flash evaporation.

2. The process as claimed in claim 1, wherein the bubble diameter of the ethylene gas is less than 1.5 mm.

3. An apparatus for carrying out the process as claimed in one of claims 1 or 2, which apparatus comprises a mixer (1) with chlorine inlet (11), a "static mixer" (2) with ethylene inlet (5), a let-down vessel (3), a recirculating pump (6) and a heat exchanger (7), the mixer (1), the "static mixer" (2), the let-down vessel (3), the recirculating pump (6) and the heat exchanger (7) being connected to each other in terms of flow, and the let-down vessel (3) being connected to a condenser (8) in terms of flow via a control valve (4), and a product discharge line (9) and a waste gas line (10) leaving the condenser (8).

4. The apparatus as claimed in claim 3, wherein a "static mixer" (2) with a flow obliquely around the web is used.

5. The apparatus as claimed in claim 4, wherein the blades in the "static mixer" (2) are perforated, slotted, serrated and/or corrugated.

6. The apparatus as claimed in claim 5, wherein the blades form meandering channels.

7. The apparatus as claimed in claim 5, wherein the blades form channels crossing each other.

8. The apparatus as claimed in claim 3, wherein a pressure-holding device (12) is arranged between the "static mixer" (2) and the let-down vessel (3).

9. The apparatus as claimed in claim 3, wherein the let-down vessel (3) is arranged 8 to 12 m above the "static mixer" (2).

## Revendications

1. Procédé de préparation d'un 1,2-dichloréthane à haute pureté avec récupération de chaleur à partir de quantités équimoléculaires d'éthylène et de chlore dans le 1,2-dichloréthane servant de solvant en présence d'un catalyseur consistant en tétrachloroferrate-I à une température de 75 à 200°C et une pression de 1 à 15 bar dans une zone de réaction, caractérisé en ce que, dans une zone de mélange préalable, on dissout le chlore gazeux dans le 1,2-dichloréthane en circulation, dans la zone de réaction placée à la suite, on fait réagir l'éthylène gazeux en phase liquide finement dispersée, à un diamètre de bulle de 2,0 mm au maximum, on fait écouler cette phase liquide finement dispersée à une vitesse de 0,3 à 1 m/s avec une durée de passage de 2,5 à 25 s, calculée sur la phase liquide, au travers de la zone de réaction puis on évacue le 1,2-dichloréthane formé, à haute pureté, contenant moins de 500 ppm de produits d'accompagnement chlorés, à l'état gazeux par vaporisation par détente.

2. Procédé selon revendication 1, caractérisé en ce que le diamètre des bulles de l'éthylène gazeux est inférieur à 1,5 mm.

3. Appareillage pour la mise en oeuvre du procédé selon une des revendications 1 ou 2, caractérisé en ce qu'il comprend un mélangeur (1) avec conduit d'injection de chlore (11), un "mélangeur statique" (2) avec un conduit d'injection d'éthylène (5), un récipient de détente (3), une pompe de circulation (6) et un échangeur de chaleur (7), le mélangeur (1), le "mélangeur statique" (2), le récipient de détente (3), la pompe de circulation (6) et l'échangeur de chaleur (7) étant reliés entre eux dans le sens de l'écoulement, le récipient de détente (3) est relié par un étranglement (4) à un condenseur (8) et ce dernier condenseur (8) est équipé d'un conduit d'évacuation du produit (9) et d'un conduit d'évacuation des gaz résiduaires (10).

4. Appareillage selon revendication 3, caractérisé en ce que l'on utilise un "mélangeur statique" (2) avec déviation du courant par des nervures obliques.

5. Appareillage selon revendication 4, caractérisé en ce que les tôles du "mélangeur statique" (2) sont perforées, fendues, dentelées et/ou ondulées.

6. Appareillage selon revendication 5, caractérisé en ce que les tôles forment des canaux sinueux.

7. Appareillage selon revendication 5, caractérisé en ce que les tôles forment des canaux qui se croisent.

8. Appareillage selon revendication 3, caractérisé en ce que, entre le "mélangeur statique" (2) et le récipient de détente (3), il y a un dispositif de retenue de la pression (12).

9. Appareillage selon revendication 3, caractérisé en ce que le récipient de détente (3) est placé 8 à 12 m au-dessus du "mélangeur statique" (2).
